# EUROPEAN PATENT SPECIFICATION

(11) **EP 1 286 658 B1**
(45) Date of publication and mention of the grant of the patent: **08.03.2006**
(21) Application number: 00992727.8
(22) Date of filing: 22.12.2000
(51) Int. Cl.: A61K 9/08, A61K 38/19

(54) **GAMMA-IFN LIQUID-DROPLET AEROSOL AND METHOD**
GAMMA-IFN FLÜSSIGTROPFENAEROSOL UND VERFAHREN
AEROSOL A GOUTTELETTES LIQUIDES DE GAMMA-IFN ET PROCEDE ASSOCIE

(30) Priority: 30.12.1999 US 173926 P
(43) Date of publication of application: 05.03.2003
(73) Proprietor: Intermune, Inc., Brisbane, CA 94005 (US)
(72) Inventor: EMLEN, J., Woodruff, Redwood City, CA 94025 (US); VAN VLASSELAER, Peter, Sunnyvale, CA 94087 (US)
(74) Representative: Vossius & Partner
(86) International application number: PCT/US2000/042831
(87) International publication number: WO 2001/049260

(56) References cited:
- EP-A- 0 257 956
- US-A- 5 780 012
- US-A- 5 971 951
- DEBS ET AL.: "Lung-specific delivery of cytokines induces sustained pulmonary and systemic immunomodulation in rats" JOURNAL OF IMMUNOLOGY, vol. 140, 1988, pages 3482-3488, XP001030667

## Description

### Field of the Invention

The present invention relates to a method of producing a liquid-droplet aerosol of human gamma interferon (γ-IFN) having a selected aerosol particle size and desired, predictable γ-IFN activity, and to an aerosol composition produced by the method.

### Background of the Invention

Although human gamma interferon (γ-IFN) has been available in isolated form for nearly twenty years, its potential in treating a variety of lung conditions, such as interstitial lung diseases, infectious diseases of the lung, airway constrictive diseases, and cystic fibrosis, and systemic conditions, such as cancer, viral or bacterial infection, and fibrotic disorders is attracting increasing attention (compare e.g. R.J.Dabs et al., "Long-specific delivery of cytokines induces sustained pulmonary and systemic immunomodulation in rats", J. of Immunology, vol. 140, 1998, pages 3482-3488). New clinical trials involving γ-IFN alone or in combination with other therapeutic compounds for the treatment of are currently in progress.

Most current γ-IFN therapies involve administering the protein by injection, e.g., by a subQ or IV route. An important perceived advantage of administration by injection is that the dose and activity of the protein can be carefully controlled. For example, the protein can be prepared in stable aqueous form, stored over extended periods without loss of activity or change in its state of aggregation, then administered in a precisely known volume.

By contrast, administering γ-IFN by inhalation, which requires aerosolizing the protein from a solution of γ-IFN, presents several challenges. In particular, it has not been known heretofore whether and how γ-IFN could be aerosolized without loss of activity and/or protein aggregation, particularly where the aerosol is formed under shear conditions necessary to produce a desired aerosol-particle size range. This uncertainty is due in part to the fact that gamma is active in a non-covalent dimeric form, but not in monomeric form. In addition, protein aggregation would be expected to reduce activity. Nor has it been known or how γ-IFN could be formulated so that its activity and molecular-size characteristics are maintained over an extended storage condition, yet still allow the desired protein properties and particle-size feature in an aerosol.

### Summary of the Invention

The present invention addresses and solves the above problems in producing a γ-IFN liquid-droplet aerosol that (i) can be produced from a storage-stable form of the protein, (ii) results in the production of aerosol particles of a desired, selected size range and size distribution, (iii) shows little if any change in γ-IFN activity, as measured by a standard test for γ-IFN activity, and (iv) shows little if any change in the molecular size distribution of the protein.

The invention includes, in one aspect, a method of administering to a selected region of a patient's respiratory tract, an amount of human gamma-interferon (γ-IFN) having a known, selected gamma-interferon biological activity and molecular size distribution. The method includes first placing an aqueous γ-IFN solution having a viscosity less than 2 Cp at room temperature, a known, selected γ-IFN biological activity, and containing a stabilizing agent and a dispersing agent, against a plate having defined-size openings. The solution is forced through the openings under conditions effective to form aqueous droplets having (a) a volume mean diameter in a selected size range of (i) less than 1 microns, (ii) 1-3 microns, (iii) 3-5 microns, (iv) 5-10 microns, (v) greater than 10 microns, or (vi) two or more of the size ranges. The droplets are characterized by a γ-IFN, biological activity and molecular size distribution substantially the same as that of the aqueous γ-IFN solution. The liquid aerosol droplets are then delivered to a patient's respiratory tract.

The biological activity of the γ-IFN in solution and droplet form may be determined by (i) the ability of γ-IFN to stimulate CD64 antigen expression in cultured enriched human monocytes, or (ii) the biological activity of the γ-IFN in solution and droplet form is determined by the ability of γ-IFN to stimulate HLA-DR antigen expression in cultured human monocytes. The γ-IFN activity of the solution is preferably at least about 1 million International units of gamma-IFN per ml, more preferably between 4 and 50 million units/ml.

An exemplary stabilizing agent is mannitol, present in an amount between 5 and 15 mM. An exemplary dispersing agent is polysorbate, present in an amount of between about 50-200 mg/liter weight percent.

In one general embodiment for forming the aerosol, the plate against which the solution is placed is vibrating at an amplitude and frequency effective to form droplets of the defined, selected size. In another general embodiment, the plate against which the solution is placed is stationary, the solution is placed against the plate under pressure, thus forming streams of extruded solution, and the streams are broken into desired, selected-size particles by passing a stream of gas over the plate.

For use in treating interstitial lung diseases, infectious diseases of the lung, bronchial constrictive diseases, and cystic fibrosis, the forming step is effective to produce droplets in the 1-3 micron size range, and may also produce droplets in the 3-5 micron size range and/or in the size range less than 1 micron.

For use in treating disease conditions that are responsive to systemically administered γ-IFN, the forming step is effective to produce droplets in the 1-3 micron size range, and may also produce droplets in the 3-5 micron size range.

In another aspect, the invention includes an aerosol composition for delivery to a patient's respiratory tract. The aerosol is formed by first placing an aqueous γ-IFN solution having a known, selected γ-IFN biological activity, a viscosity less than 2 Cp at room temperature, and containing a stabilizing agent and a dispersing agent, against a plate having defined-size openings. The solution is forced through the openings under conditions effective to form aqueous droplets having (a) a volume mean diameter in a selected size range of (i) less than 1 microns, (ii) 1-3 microns, (iii) 3-5 microns, (iv) 5-10 microns, (v) greater than 10 microns, or (vi) two or more of the size ranges. The droplets are characterized by a γ-IFN biological activity and molecular size distribution substantially the same as that of the aqueous γ-IFN solution.

The composition may contain at least one million International units of gamma-IFN/ml, preferably 4-50 million IU/ml, as measured by (i) the ability of γ-IFN to stimulate CD64 antigen expression in cultured enriched human monocytes, or (ii) the biological activity of the γ-IFN in solution and droplet form is determined by the ability of γ-IFN to stimulate HLA-DR antigen expression in cultured human monocytes.

These and other objects and features of the invention will become more fully apparent from the following detailed description of the invention, when read in conjunction with the accompanying drawings.

### Brief Description of Drawings

Figs. 1A and 1B are cross-sectional views, in simplified form, of an apparatus useful in practicing the method of the invention, shown in storage (1A) and operative (1B) states;
Figs. 2A and 2B are cross-sectional views, in simplified form, of another apparatus useful in practicing the method of the invention, shown in storage (2A) and operative (2B) states;
Figs. 3A and 3B are plots showing the correlation in flow rates (3A) of a γ-IFN solution vs. saline solution in an aerosolization apparatus operated at two different power levels and volume mean diameters (VMD) of aerosol liquid droplets formed by the aerosolization apparatus at the two different power (3B);
Fig. 4 is a plot showing the correlation between mean aerodynamic diameters (VMD) of saline and γ-IFN solution and the percentage fine particle fraction (%FPF), defined as the percentage of particles in the 0.5 to 6.0 micron size range, formed by the above aerosolization device at two different power levels for a γ-IFN solution and saline solution;
Figs. 5A and 5B are plots of flow rates as a function of VMD (5A) or %FPF (5B) at high and low power levels of the aerosolization device for a γ-IFN solution and saline solution;
Fig. 6 shows plots of CD64 antigen expression in monocytes stimulated with (i) increasing dilutions with each of three different γ-IFN solutions after aerosolization (solid diamonds, solid squares, solid triangles), (ii) the same γ-IFN solution, but without aerosolization (x's) and (iii) medium alone;
Figs. 7A and 7B are liquid chromatograph profiles of control (pre-aerosolized) (8A) and aerosolized (8B) γ-IFN solution; and

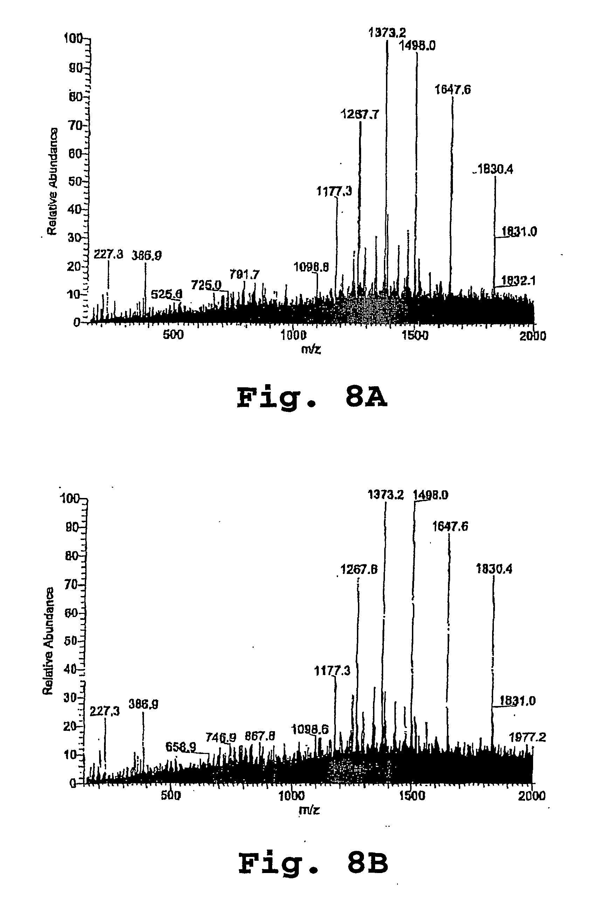

Figs. 8A and 8B are mass spectrographs of the region of the 13.77 peak in the Fig. 7A and 7B chromatographs, respectively.

### Detailed Description of the Invention

### A. Definitions

Unless otherwise indicated, the terms herein have the following meanings:
"Human gamma-interferon" or "γ-IFN" refers to a polypeptide having the activity of human γ-IFN, as defined and disclosed in U.S. Patent No. 5,096,705. The peptide may have the full native human γ-IFN sequence, as disclosed in U.S. Patent No. 5,096,705, or have N-terminal and/or C-terminal truncations of the full-length native peptide, as disclosed, for example, in U.S. Patent Nos. 5,690,925, 5,582,824, 5,574,137, 4,921,698, 4,832,959, and 4,604,284, or contain internal deletion or substitutions mutations, such as disclosed in U.S. Patent No. 4,845,196.
"γ-IFN solution" refers to a solution of γ-IFN containing stabilizing and dispersing agents, preferably having a γ-IFN biological activity of at least 1 million International units (IU or units) of gamma-IFN per ml, more preferably between 4 and 50 million units/ml, corresponding to 0.05 to 2.5 mgs/ml γ-IFN. One preferred γ-IFN composition, referred to as γ-IFN Solution 1, has the composition shown in Table 1 below. This composition is sold under the trademark of ACTIMMUNE, and includes recombinant human interferon γ-1 B, rhlGB, as disclosed and claimed in US Patent No. 5,582,824.
An "aerosol" or "liquid-droplet aerosol" is a suspension of liquid-droplet particles in a gaseous medium, e.g. air. An "aqueous aerosol" is an aerosol formed from an aqueous solution, meaning one that contains at least about 90% water, preferably 5% or more water as the solution solvent
"Mean particle size" or "volume mean aerodynamic diameter (VMD)" refers to the arithmetic average size of particles in a particle aerosol, as determine by standard particle-sizing methods. The particles making up an aerosol composition preferably have a narrow particle distribution, e.g., less than 1-2 standard deviations from the mean. Altematively, 80%, and preferably 95% of the particles have a size in a selected size range, e.g., 1-3 microns.
"% FPF (0.5-6.0 µm)" is percentage fine particle fraction with volumetric diameters between 0.5 and 6.0 µm. This size range as measured by laser ensemble light scattering is the fraction suitable for pulmonary deposition.
"Flow Rate" is aerosol production rate measured in microliters aerosolization per second (µL/s).
"A pulmonary disease condition" is a pulmonary condition characterized by excess fibrosis or collagen formation, or by inadequate macrophage infiltration at a pulmonary site, particular in infection. The term includes, without limitation, interstitial pulmonary fibrosis, tuberculosis, viral or bacterial pneumonia, and chronic granulomatous disease.
"An amount of the aerosol effective to deliver at least about X µg of human γ-IFN to the patient's lungs" is the amount of human γ-IFN in an aerosol composition needed to deliver a desired amount of human γ-IFN to the patient's lungs, and is related to the efficiency of aerosol delivery, that is, the percentage of human γ-IFN that reaches the lungs/total amount of human γ-IFN in the aerosol. For example, to administer 50 µg of human γ-IFN to the patient's lungs, where the efficiency of delivery is 50%, a total aerosol amount of 100 µg of human γ-IFN is administered.

### II. Aerosolization Method

The invention includes, in one aspect, a method of forming an aqueous aerosol of γ-IFN, for use in administering a selected dose of γ-IFN to a subject, e.g., human subject. As noted earlier, the method will be able to realize its potential over other forms of γ-IFN delivery only if the following objectives can be reliably attained: (i) long storage time with little or no loss of γ-IFN biological activity, (ii) reproducible production of aerosol liquid droplets within a well-defined size range or size ranges, (iii) an amount of γ-IFN in the aerosol sufficient for therapeutic effectiveness, and (iv) little or no loss of biological activity or change in the molecular state of the γ-IFN. This section discusses the novel composition and aerosolization conditions that have been found, in accordance with the invention, to achieve these goals.

### A. γ-IFN Solution

According to one aspect of the invention, it has been discovered that a stable γ-IFN solution containing γ-IFN at a concentration of at least 1 million International units (IU or units) of gamma-IFN per ml, more preferably between 4 and 50 million units/ml, can be aerosolized into well-defined droplet sizes, and with little or no loss of biological activity or change in molecular size distribution of γ-IFN, which is a dimer in its native, active state. That is, the molecule is neither monomerized nor aggregated in the aerosolization step. The stated concentrations allow, for example, a dose as great as 1 million units in an aerosol volume as small as 25 microliters.

In addition of low-concentrations of buffer components, which maintain the solution at a pH preferably of about 5.5 ± 0.5, the solution contains a stabilizing agent which helps to stabilize the protein on storage in solution and a dispersing agent which helps maintain the protein in a monodisperse form in solution, and/or assist in solubilizing the solution components upon rehydration from a dried, e.g., lyophilized state.

The stabilizing agent is preferably a sugar, such as mannitol, lactose, maltose, dextrose, sorbitol, glucose, or dextrose, an alcohol, such as glycerol, ethylene glycol in monomer or polymer form, or amino acid. The stabilizing agent is preferably present in an amount of about 1-10 g/liter, or alternatively, in a molar amount of between 2-25 mM. One preferred stabilizing agent is mannitol, present in an amount between 5-15 mM.

The dispersing agent is preferably a nonionic surfactant, such as a polysorbate, preferably Polysorbate 20 or Polysorbate 80, or a polysaccharide, such as carboxymethylcellulose, or alginate. The dispersing agent is preferably present in an amount between 20-500 mg/liter. One preferred dispersing agent is Polysorbate 80, present in an amount of between about 50-200 mg/liter. In particular, the dispersing agent should be present in an amount that increases the viscosity to no more than about 10, preferably no more than about 6 Cp at 25°C.

The solvent solution is water or water with relatively small amounts, e.g., less than 5-10 volume percent, of a water-miscible solvent, such as ethanol. One disadvantage of more volatile solvents in the solution is that rapid evaporation during and after aerosolization can cause significant reduction in droplet size.

Table 1 below gives the composition for one preferred of γ-IFN solution, referred to herein as γ-IFN Solution 1, and containing about 4 million units γ-IFN/ml. The solution provides storage at 4°C for up to several months without appreciable loss of γ-IFN activity or change in the molecular aggregation state of the protein.

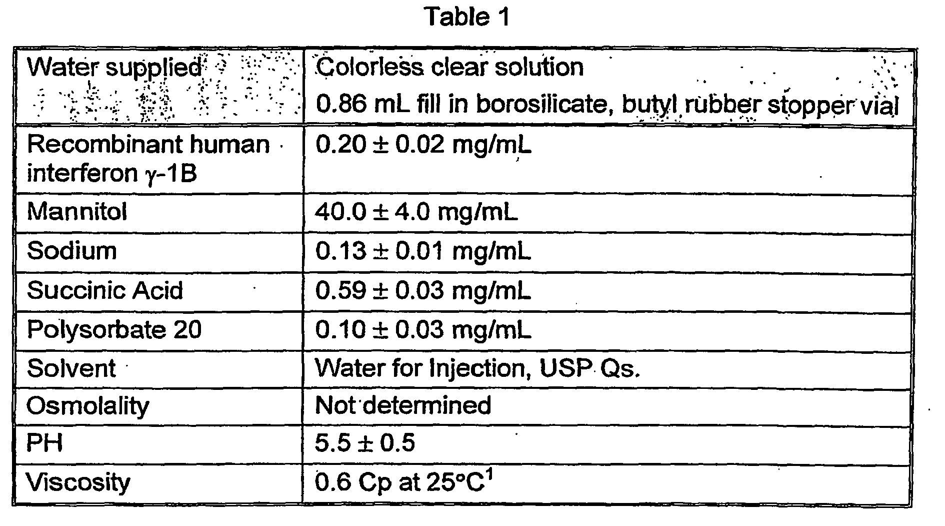

### B. Aerosol Production

According to an important feature of the invention it has been discovered that a γ-IFN solution formulated as above can be aerosolized under conditions that produce defined-size droplet particles in a selected size range such as (i) less than 1 micron, (ii) 1-3 microns, (iii) 3-5 microns, (iv) 5-10 microns, (v) greater than 10 microns, or (vi) two or more of these size ranges, while producing little or no loss of biological activity and little or no change in molecular distribution state.

The method preferably includes first placing the γ-IFN solution against a plate having defined-size openings or pores. The pore sizes are preferably straight-through, (constant-diameter) pores having selected sizes in the 0.5 to 10 micron size range. In general, the size of the aerosol particles produced are directly related (though not necessarily identical) to plate pore size. The other important variable is flow rate of solution through the plate, which will depend both on the viscosity of the solution, and the "pressure" applied to the solution. As will be seen below, this pressure can be either actual hydrostatic pressure or, where droplets are formed by movement through a vibrating plate, as considered below) the frequency and driving amplitude of the plate.

The solution is forced through the openings under conditions effective to form aqueous droplets having a volume mean diameter in one or more of the above selected size ranges. The droplets are characterized by a γ-IFN biological activity and molecular size distribution substantially the same as that of the aqueous γ-IFN solution. The liquid aerosol droplets are then delivered to a patient's respiratory tract.

One preferred device for carrying out the method is illustrated in Figs, 1A and 1B, and is detailed in U.S. Patent No. 5,971,951, incorporated herein by reference. With reference to the figures, the device, illustrated at 10, includes an aerosol chamber 12 that terminates at its left end in the figure in a mouthpiece 14. A drug-delivery container 16 is partially filled with a γ-IFN solution 19 in accordance with the invention. The container has compressible or collapsible walls 18 and a bottom wall 21 against which a force is applied in the aerosolization procedure.

The container is fitted with a porous, low-resistance filter 20 which allows solution to flow freely through it, but serves to prevent passage of any particulate matter in the solution. Between the container and chamber 12 is a porous membrane plate 22 having formed therein, a plurality of pores, such as pores 24 through which solution is forced during aerosolization. The filter plate is preferably formed of a material, such as polycarbonate or polyester, having a density of about 1.7 mg/cm², and a thickness between about 8-12 microns.

The pore sizes (diameters) in plate 22 are roughly 1/2 the desired aerosol particle diameters. Thus, for example, to generate an aerosol with particles in the 1-3 micron range, a membrane plate with pore diameters of between about 0.5 and 1.5 would be selected. However, particle size is also responsive to the rate of flow through the plate, which in turn is determined both by the viscosity of the solution, and the force applied to the container.

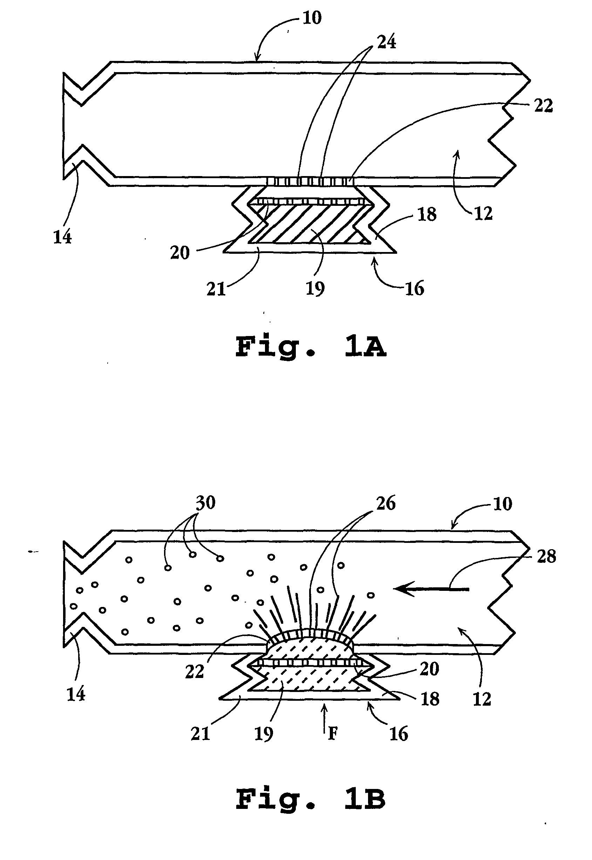

The force that is applied to the container, to force solution 19 though the filter and then extrude the solution through the pores in plate 22 is preferably one that produces a fluid pressure of about 50 bar or less, more preferably 35 bar or less. As seen in Fig. 1 B the application of pressure to the container forces solution through the membrane plate, in effect, forming extruded streams of liquid, such as indicated at 26, which are broken up into droplets 30, in part by the movement of airflow through the chamber in the direction of arrow 28.

As seen in Fig. 1B, device 10 is designed such that the force applied to the container acts to deform the plate inwardly, positioning the outer surface of the plate more toward the center region of chamber 12 to place the outer surface of the plate in the center region of chamber 12, such that the extruded solution is entrained in the faster moving air close near the center of the chamber.

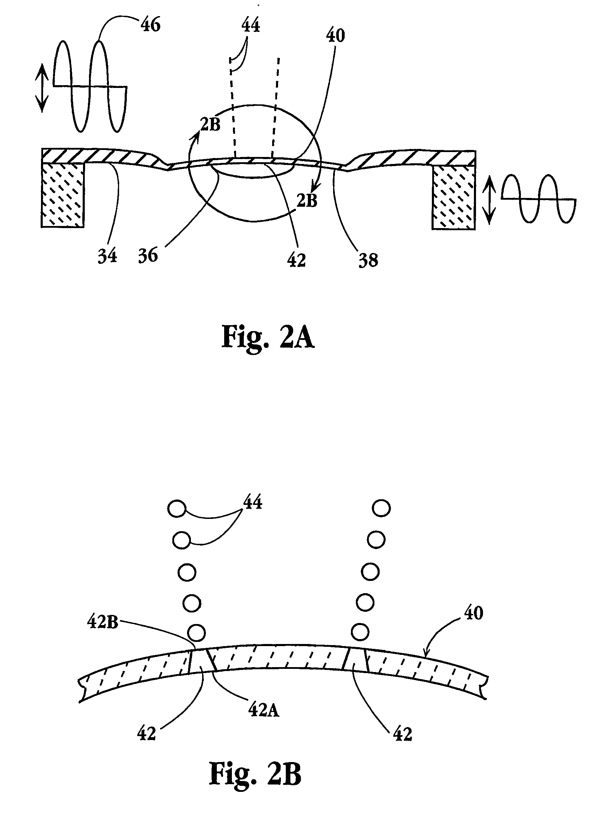

Fig. 2B is a cross-sectional view of the critical aerosol-generating components of another type of aerosolization device 34 useful in forming a γ-IFN aerosol having desired-size droplets and with little or no loss of γ-IFN activity or change in protein molecular-size distribution. Device 34 is detailed, for example, in U.S. Patent No. 5,938,117, which is incorporated herein by reference. The device includes a container (not shown) which holds the γ-IFN solution, and which supplies the solution in a pool 36 formed on the inner surface 38 of a vibrating membrane 40.

As seen on Fig. 2b, membrane 40 has a plurality of conical openings or pores, such as pore 42, which are shaped and dimensioned to produce desired-size droplets, such as 44, when the plate is vibrated at a certain amplitude and frequency. Plate vibration is produced by an oscillator (indicated by wave 46), such as a piezoelectric oscillator that oscillates at a frequency typically in the 50-80K Hz range. The vibrating plate produces, in effect, surface pressure which cause the solution pooled on the plate to move through the pores and break off into desired size particles. The size droplets formed by the device, as well as the rate of droplet ejection, depends on the size of the plate pores, amplitude and frequency of vibration, and the viscosity of the solution being aerosolized. In general, and as indicated in Fig. 2B, droplet size is between the smaller and larger pore openings. For example, to produce droplet particles in the 1-2 micron size range, the larger pore opening (42A) is about 1.5 microns, and the smaller opening (42B), about 0.6-0.7 microns.

### C. Aerosol processing variables

The uniformity, predictability, and size distribution of droplet particles formed in accordance with the invention is sensitive to solution viscosity, which will affect the flow rate of solution in the aerosolizing system, and the presence of certain components, such as surfactants and proteins, which can affect surface tension properties of the solution.

Aerosolizing systems, such as those described above, are typically calibrated with a standard dilute, e.g., 10mM saline solution, that have viscosity and surface tension properties close to water. Using a standard saline solution, droplet sizes and size distributions can be measured for particular processing parameters, particularly flow rate and plate pore size. To establish that a given solution will give predictable droplet size and size distribution characteristics, at known processing parameters (flow rate and pore size), it is therefore necessary to establish that the solution behaves substantially like dilute saline in the aerosolizing system.

In the present case, a γ-IFN solution identified as Solution 1 and having the components given in Table 1, was compared against a 10mM saline solution in an Aerogen aerosolizer (AG) as detailed in Example 1. Briefly, flow rates, VMDs, and %FPF were measured for the two solutions in each of six different devices, and at each of two different device power settings.

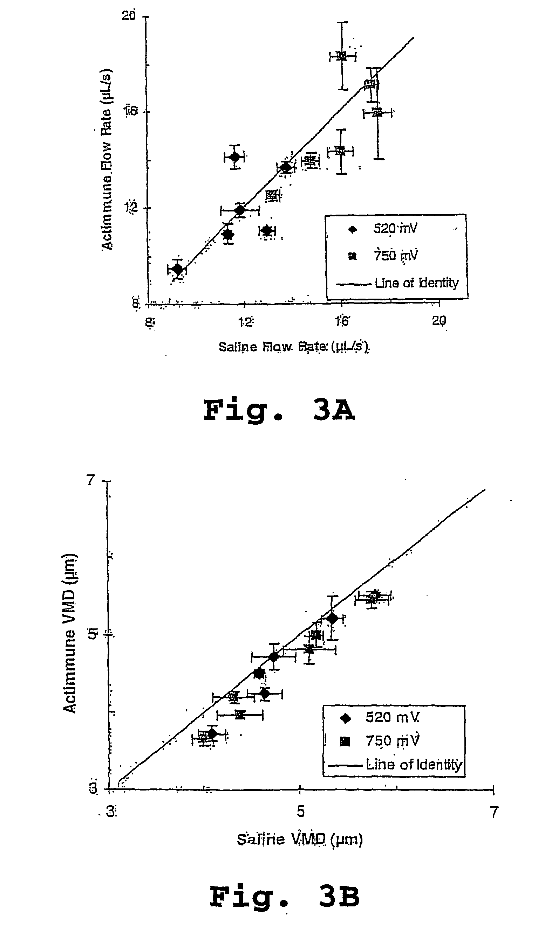

Flow rates were determined for both test solutions at low (520 mV) and high (750 mV) power, with the results shown in Fig. 3A. Each point represents an AG at lower or high power. Flow rates ranging from 9 ul/s to 18 uL/sec were recorded. Solution 1 had a mean decline of 0.4 uL/sec compared to saline, but the decrease is both functionally negligible and not statistically significant. Inspection of the figure show that flow rates increased at high power.

The VMDs of saline and Solution 1 were compared using the same six AG's, which were designed to produce VMDs in particle size ranges between about 3.5 and 5.5 microns. Fig. 3B shows that Solution 1 VMDs are slightly below the line of identity, and therefore slightly smaller than the corresponding saline-solution values. At low power setting, the mean difference in VMD between the Solution-1 droplets and saline droplets was about 0.2 micron, and at the high power setting, about 0.3 microns. The slight reduction in VMD increases the %FPF slightly.

Like the saline solution, Solution 1 showed no significant difference in VMD at low and high power settings, demonstrating that the power can be changed to increase aerosolization rate without significantly affecting mean droplet size.

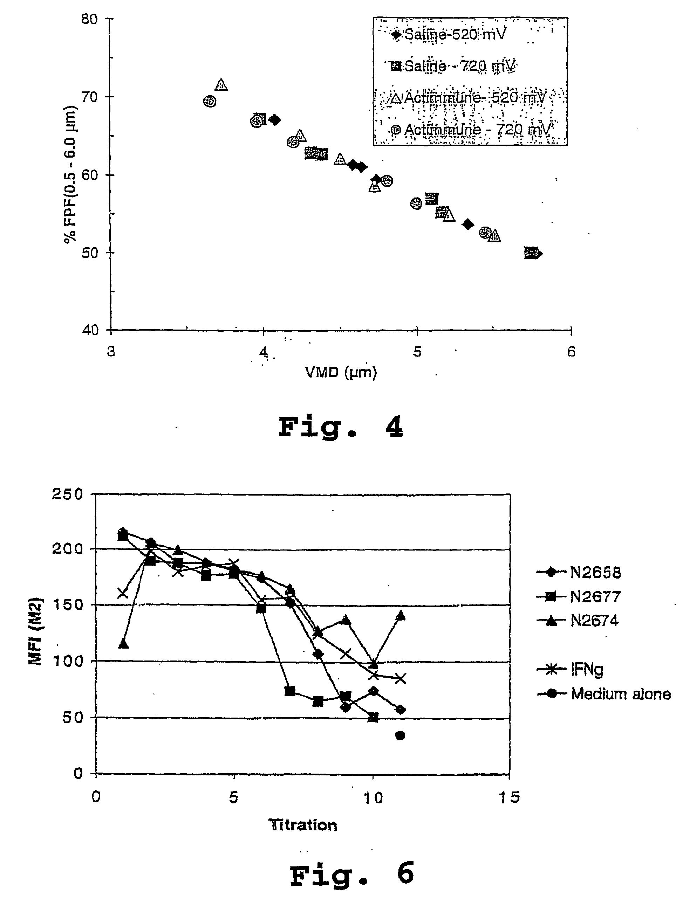

VMD and 5FPF (% particles in the 0.5 to 6.0 micron size range) were strongly correlated for both solutions, as seen in Fig. 4. The data indicate the very nearly identical behavior in particle size and size distribution attained with each of the two solutions.

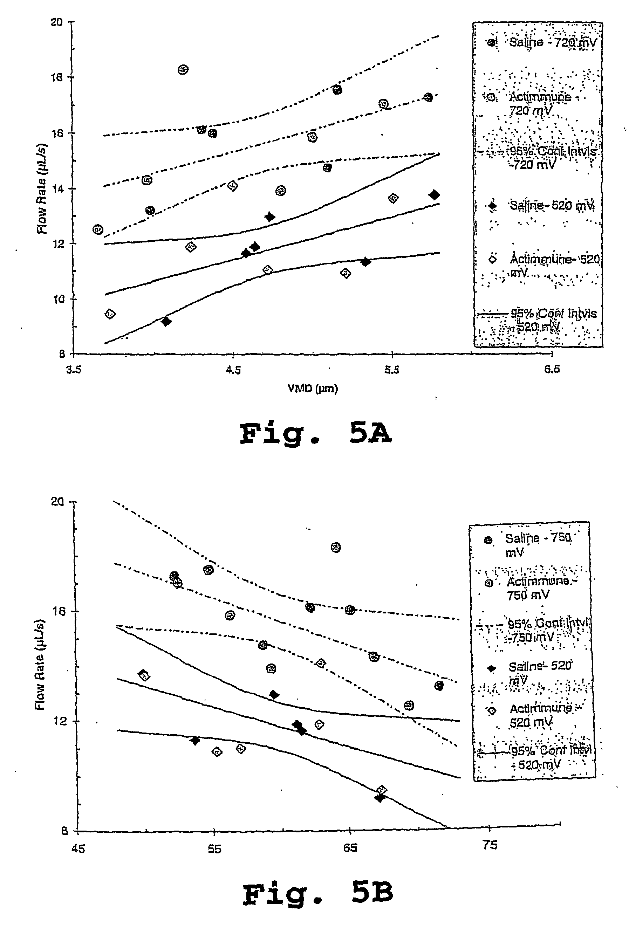

Since no significant differences between formulations at a given power level were observed, the data were pooled and linear regressions calculated. The coefficients were found to be statistically significant at P ≤ 0.05. Flow rates were proportional to VMD (Fig. 5A). There was no statistical differences between the slopes at high/low power detected. Within the VMD range of interest, flow rate increased approximately 1.5 uL/s for each u< VMD increase. As can be expected from the inverse relationship between VMD and %FPF (Fig. 4), %FPF increased with decreasing flow rate (Fig. 5B).

The studies above demonstrate a γ-IFN solution prepared in accordance with the invention has substantially the same flow rate, droplet size, and droplet size distribution characteristics as a standard dilute saline solution, when aerosolized in accordance with the invention.

### D. Aerosol characteristics

In addition to predictability and uniformity in droplet size, it is important that the aerosolization process, which is associated with high local shear forces, does not significantly alter the biological activity or the molecular size distribution of γ-IFN in the aerosol. The two characteristics may be linked, inasmuch as γ-IFN is active in a dimerized state, and may be expected to lose activity, either by monomerization of aggregation, when subjected to the high shear forces associated with aerosolization, and/or by protein denaturation at the liquid/air interfaces in small aerosol droplets.
The biological activity of a γ-IFN solution, before and after aerosolization, may be assessed by a standard in vitro methods for assaying γ-IFN activity. For example, the biological activity of the γ-IFN in solution and droplet form can determined by either (i) the ability of γ-IFN to stimulate CD64 antigen expression in cultured enriched human monocytes, or (ii) the ability of γ-IFN to stimulate HLA-DR antigen expression in cultured human monocytes.

The former method was used to demonstrate that aerosolization in accordance with the invention has little or no effect on biological activity of the IFN. Briefly, human monocytes were stimulated in culture with decreasing concentrations of γ-IFN solution, either in non-aerosolized form, or after aerosolization. The cells were cultured in standard medium overnight at 37°C. FITC-labeled antiCD64 was then added to the cells, and after cell washing to move unbound antibody, fluorescence associated with the cells was determined.

Results are shown in Fig. 6 for three representative aerosolized formulations (solid diamonds, solid squares, and solid triangles), γ-IFN Solution 1 prior to aerosolizing (star symbols), and medium alone (solid circles). As seen, the three aerosolized formulations showed substantially the same biological activity as the non-aerosolized solution. Medium alone gave the expected low induction of CD64 surface antigen.

The results demonstrate that a γ-IFN solution prepared in accordance with the invention, when aerosolized in accordance with the invention, gives little or no loss in biological activity of the γ-IFN.

To determine the effect of aerosolization on the molecular size distribution of γ-IFN, γ-IFN solution before and after aerosolization was analyzed by liquid chromatography, to identify and isolate the basic γ-IFN peak, followed by mass spectroscopy to identify different size/charge species within the γ-IFN peak.

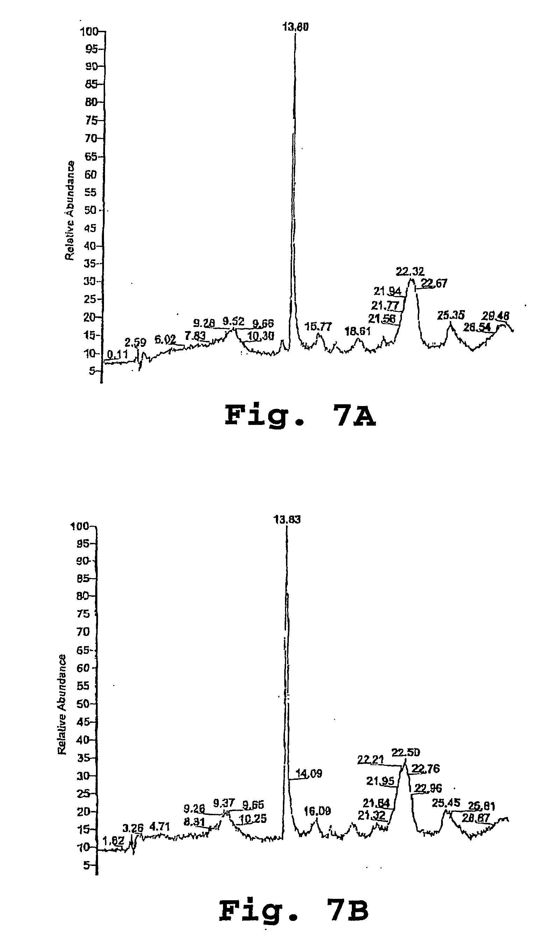

Figs.7A and 7B are chromatograms of γ-IFN Solution 1 fractionated by liquid chromatography, before and after aerosolization in accordance with the invention. The two chromatograms are essentially identical, having a single γ-IFN peak at about 13.80, corresponding to dimerized γ-IFN and two other strong peaks at about 9.52 and 22.32 corresponding to polysorbate peaks.

The γ-IFN peaks from each sample were isolated and further analyzed by mass spectroscopy, with the results shows in Figs, 8A and 8B. The several dominant peaks between 1177 and 1830 correspond to various protonated forms of a single dimeric species.

Collectively, the molecular analysis of the samples shows that aerosolization of the γ-IFN solution has no measurable effect on the molecular size distribution, i.e., state of dimerization or aggregation of the γ-IFN. It was also confirmed that exposing the γ-IFN solution to the aerosol metal plate overnight had no effect on the molecular distribution of the compound.

### III Use of the aerosol in therapy

In accordance with the invention, the liquid-droplet human γ-IFN aerosols produced as above are used in treating a variety of pulmonary conditions, by direct γ-IFN delivery to the affected area of the respiratory tract, or for delivery of γ-IFN to the bloodstream, for treating systemic conditions, such as viral infection, cancer, or fibrotic conditions, responsive to systemically administered γ-IFN.

In most cases, the desired droplet particle size of the aerosol will be 1-3 micron, for delivery to bronchial sites. In disease states, such as cystic fibrosis, where there may be little air movement in the lower respiratory tract, even small sizes, e.g., less than 1 micron, may be desired. Where other areas of the respiratory tract are affected, or where it is desired to administer γ-IFN systemically, larger particle sizes, typically 3-5 microns, or a combination of small and larger particles, e.g., 1-3 microns and 3-5 microns, may be most effective.

The delivered dose of γ-IFN is preferably at least 10 µg (200,000 units), and may be as high as 200 µg (4 million units) or more. Typical aerosol volumes are 50-200 ul, with about 50-80% of the total aerosol volume reaching the target area. The dosing schedule is typically 2-3 times a week, for a period required to achieve the therapeutic objective.

As examples of conditions that can be treated, a variety of pulmonary disease conditions are characterized by excess fibrosis or collagen formation, or by inadequate macrophage infiltration at a pulmonary site, particularly in infection. For some disease conditions, e.g., interstitial pulmonary fibrosis (IPF), the therapeutic end point may require dosing for 6-12 months or longer, or over an extended period as long as long as the disease state needs controlling. For other conditions, e.g., bacterial or viral infection, the dosing is repeated until the infection, or the symptoms of the infection, have cleared.

One advantage of the present method over injection forms of human γ-IFN treatment is that relatively high doses, e.g., in the range 200-500 µg doses may be administered without the serious side effects seen at the upper limits of tolerance of injected γ-IFN, which is about 250 µg. The therapy, e.g., in the treatment of IPF, may be carried out in combination with other treatment methods, e.g., corticosteroids (prednisolone 10 mg/day or less), non-steroidal anti-inflammatory drugs, and analgesics. One preferred combination therapy for IPF is human γ-IFN in combination with prednisolone, given orally, 10 mg/week or less.

For treating drug-resistant tuberculosis, therapy may be carried out in combination with drugs currently used in treating tuberculosis, such as clofazimine, ofloxacin or rifabutin, where the latter drug is given at normal or reduced dosages. The therapy is continued until the infection is controlled, e.g., no further signs of active infection.

For treating viral or bacterial pneumonia, the amount of human γ-IFN administered per dose may be as little as 10 µg or as high as 200-300 µg, administered at least twice but typically for no more than a week. The therapy may be carried out in combination with drugs currently used in treating viral or bacterial infections.

For treating chronic granulomatous disease, the aerosolized compound is administered at least 2-3 times per week, over a period of at least 6 months and typically for as long as the condition exists and is in need of control. Similar regimens are suitable for treating cystic fibrosis (CF) and asthma, although in the latter condition, a combination of small and larger droplet sizes may be preferred.

From the foregoing, it can be appreciated how various objects and features of the invention are met. The aerosolizing method is effective to produce a γ-IFN an aerosol that has predictable droplet sizes and size distribution, in desired size ranges that are efficiency targeted to selected regions of the respiratory tract Accordingly, high drug-dose efficiency, between 50-80%, can be achieved, allowing for effective dosing with relatively little loss of protein material, and in predictable doses.

The γ-IFN dose can be stored, e.g., in liquid form, for extended periods, with little loss of activity for periods of several months or more, and this solution can be aerosolized, in accordance with the invention, with little or no loss in biological activity or size distribution of the protein.

As a result, the advantages of inhalation therapy over other forms of parenteral administration, usually involving needle injection, are achieved while retaining relatively precise drug doses and drug-delivery efficiency.

The following examples illustrate methods of measuring performance target specifications in accordance with the invention. The examples are intended to illustrate the invention.

### Example 1

### Aerodynamic Study Methods

Aerosol generators (AG) were obtained from Aerogen, Inc., (Sunnyvale, CA). Specifically, six AGs designed to produce different droplet sizes in the range approximately 3.5-5.0 microns, were selected and the VMDs, the % FPF, and flow rates were measured in 10-mM saline and γ-IFN Solution 1 at low end (520 mV) and high end (750 mV) power settings. The means and sample standard deviation of replicate determination were tabulated. Table 2 shows the target specifications of all the performances.

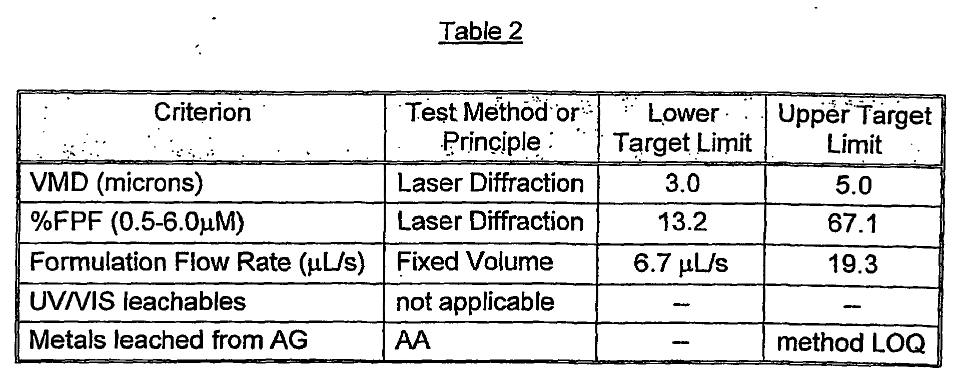

Table 3 below shows the analytical methods used for measuring various bulk-phase and droplet characteristics.

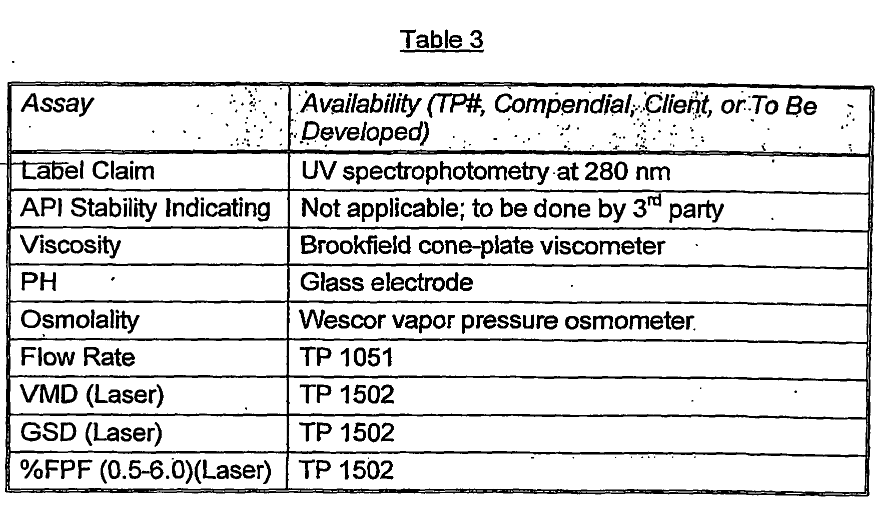

While the invention has been described with respect to particular embodiments, it will be appreciated that various changes may be made without departing from the claimed invention.

## Claims

1. A liquid-droplet aerosol composition for delivery to a patient's respiratory tract
(a) formed by placing an aqueous γ-IFN solution having a known, selected γ-IFN biological activity, and containing a stabilizing agent and a dispersing agent, against a plate having defined-size openings, and forcing the solution through said openings under conditions effective to form aqueous droplets,
(b) **characterized by** an aerosol of aqueous droplets having
(1) a volume mean diameter in a selected size range selected from the group consisting of
(i) less than 1 micron,
(ii) 1-3 microns,
(iii) 3-5 microns,
(iv) 5-10 microns,
(v) greater than 10 microns, and
(vi) two or more of the size ranges,
(2) a γ-IFN biological activity substantially the same as that of the solution, and
(3) a γ-IFN molecular size distribution substantially the same as that of the solution.

2. The composition of claim 1, wherein said solution contains at least one million International units of gamma-IFN/ml, as measured by
(i) the ability of γ-IFN to stimulate CD64 antigen expression in cultured enriched human monocytes, or
(ii) the ability of γ-IFN to stimulate HLA-DR antigen expression in cultured human monocytes.

3. The composition of claim 1 or 2, wherein said solution includes mannitol as a stabilizing agent.

4. The composition of claim 3, wherein said mannitol is present in an amount between 5-15 mM.

5. The composition of any one of claims 1 to 4, wherein said solution includes polysorbate as a dispersing agent.

6. The composition of claim 5, wherein the polysorbate is present in an amount between 50-200 mg/liter.

7. The composition of any one of claims 1 to 6, wherein the solution has a viscosity at room temperature of less than 2Cp.

8. A process for the preparation of a liquid droplet aerosol composition as defined in any one of claims 1 to 7, the composition having a known, selected gamma-interferon biological activity and molecular size distribution, whereby the process comprises:
placing an aqueous human γ-IFN solution against a plate having defined-size openings, the solution having a known, selected γ-IFN biological activity, and containing a stabilizing agent and a dispersing agent;
forcing the solution through said openings under conditions effective to form aqueous droplets having
(a) a volume mean diameter in a size range of
(i) less than 1 micron,
(ii) 1-3 microns,
(iii) 3-5 microns,
(iv) 5-10 microns,
(v) greater than 10 microns, or
(vi) two or more of i-v;
(b) a γ-IFN biological activity substantially the same as that of the solution, and
(c) a γ-IFN molecular size distribution substantially the same as that of the solution.

9. Use of the liquid droplet aerosol composition of any one of claims 1 to 7 for the preparation of a pharmaceutical composition for treating pulmonary disorders, viral or bacterial infection, cancer, or fibrotic disorders.

10. The use according to claim 9, for treating viral or bacterial pneumonia, drug-resistant tuberculosis, chronic granulomatous disease, cystic fibrosis, or asthma.

## Patentansprüche

1. Flüssigkeitströpfchen-Aerosol-Zusammensetzung zur Abgabe an die Atemwege eines Patienten
(a) die **dadurch** erzeugt wird, dass eine wässrige γ-IFN-Lösung, die eine bekannte, ausgewählte γ-IFN-biologische Aktivität hat und einen Stabilisator und ein Dispersionsmittel enthält, gegen eine Platte platziert wird, die Öffnungen von definierter Größe hat, und die Lösung unter Bedingungen, die geeignet sind, wässrige Tröpfchen zu bilden, durch die Öffnungen gepresst wird,
(b) **gekennzeichnet durch** ein Aerosol von wässrigen Tröpfchen, die
(1) einen mittleren Volumendurchmesser in einem ausgewählten Größenbereich haben, der ausgewählt wird aus der Gruppe bestehend aus
(i) kleiner als 1 Mikron,
(ii) 1-3 Mikron,
(iii) 3-5 Mikron,
(iv) 5-10 Mikron,
(v) größer als 10 Mikron, und
(vi) zwei oder mehrere der Größenbereiche,
(2) eine γ-IFN-biologische Aktivität, die im wesentlichen die gleiche ist wie die der Lösung, und
(3) eine γ-IFN-Molekülgrößenverteilung, die im wesentlichen die gleiche ist wie die der Lösung.

2. Zusammensetzung nach Anspruch 1, wobei die Lösung zumindest eine Million internationaler Einheiten des gamma-IFN/ml enthält, gemessen durch
(i) die Fähigkeit von γ-IFN, CD64-Antigenexpression in gezüchteten, angereicherten menschlichen Monocyten zu stimulieren, oder
(ii) die Fähigkeit von γ-IFN, HLA-DR-Antigenexpression in gezüchteten menschlichen Monocyten zu stimulieren.

3. Zusammensetzung nach Anspruch 1 oder 2, wobei die Lösung Mannit als Stabilisator enthält.

4. Zusammensetzung nach Anspruch 3, wobei das Mannit in einer Menge zwischen 5-15 mM vorhanden ist.

5. Zusammensetzung nach einem der Ansprüche 1 bis 4, wobei die Lösung Polysorbat als ein Dispersionsmittel enthält.

6. Zusammensetzung nach Anspruch 5, wobei das Polysorbat in einer Menge zwischen 50-200 mg/Liter vorhanden ist.

7. Zusammensetzung nach einem der Ansprüche 1 bis 6, wobei die Lösung bei Raumtemperatur eine Viskosität von weniger als 2 Cp hat.

8. Verfahren zur Herstellung einer Flüssigkeitströpfchen-Aerosol-Zusammensetzung wie in einem der Ansprüche 1 bis 7 definiert, wobei die Zusammensetzung eine bekannte, ausgewählte γ-Interferon-biologische Aktivität und Molekülgrößenverteilung hat, wobei das Verfahren umfasst:
Platzieren einer wässrigen γ-IFN-Lösung, die eine bekannte, ausgewählte γ-IFNbiologische Aktivität hat, gegen eine Platte, die Öffnungen von einer definierten Größe hat, wobei die Lösung eine bekannte, ausgewählte γ-IFN-biologische Aktivität hat und einen Stabilisator und ein Dispersionsmittel enthält;
Pressen der Lösung durch die Öffnungen, unter Bedingungen, die geeignet sind, wässrige Tröpfchen zu bilden, die
(a) einen mittleren Volumendurchmesser in einem Größenbereich haben von
(i) kleiner als 1 Mikron,
(ii) 1-3 Mikron,
(iii) 3-5 Mikron,
(iv) 5-10 Mikron,
(v) größer als 10 Mikron, oder
(vi) zwei oder mehrere von i-v;
(b) eine γ-IFN-biologische Aktivität, die im wesentlichen die gleiche ist wie die der Lösung, und
(c) eine γ-IFN-Molekülgrößenverteilung, die im wesentlichen die gleiche ist wie die der Lösung.

9. Verwendung der Flüssigkeitströpfchen-Aerosol-Zusammensetzung nach einem der Ansprüche 1 bis 7 für die Herstellung eines Arzneimittels zur Behandlung von Lungenkrankheiten, viralen oder bakteriellen Infektionen, Krebs oder fibrotischen Erkrankungen.

10. Verwendung nach Anspruch 9, zur Behandlung viraler oder bakterieller Pneumonie, arzneimittelresistenter Tuberkulose, chronischer Granulomatose, zystischer Fibrose oder von Asthma.

## Revendications

1. Composition d'aérosol à gouttelettes liquides pour la distribution aux voies respiratoires d'un patient
(a) formée en plaçant une solution aqueuse de IFN-γ ayant une activité biologique IFN-γ connue et sélectionnée, et contenant un agent stabilisant et un agent dispersant, contre une plaque ayant des ouvertures de taille définie, et en forçant la solution à passer au travers desdites ouvertures dans des conditions efficaces pour former des gouttelettes aqueuses,
(b) **caractérisée par** un aérosol de gouttelettes aqueuses ayant
(1) un diamètre volumique moyen dans une gamme de taille sélectionnée choisie dans le groupe constitué par
(i) moins de 1 microns,
(ii) 1-3 microns,
(iii) 3-5 microns,
(iv) 5-10 microns,
(v) plus de 10 microns, et
(vi) deux ou plus des gammes de taille,
(2) une activité biologique IFN-γ qui est essentiellement la même que celle de la solution, et
(3) une distribution de taille moléculaire de IFN-γ qui est essentiellement la même que celle de la solution.

2. Composition selon la revendication 1, dans laquelle ladite solution contient au moins un million d'unités Internationales de IFN-gamma/ml, comme mesuré par
(i) la capacité de IFN-γ à stimuler l'expression de l'antigène CD64 dans des monocytes humains enrichis cultivés, ou
(ii) la capacité de IFN-γ à stimuler l'expression de l'antigène HLA-DR dans des monocytes humains cultivés.

3. Composition selon la revendication 1 ou 2, dans laquelle ladite solution comprend du mannitol comme agent stabilisant.

4. Composition selon la revendication 3, dans laquelle ledit mannitol est présent dans une quantité comprise entre 5 et 15 mM.

5. Composition selon l'une quelconque des revedications 1 à 4, dans laquelle ladite solution comprend du polysorbate comme agent dispersant.

6. Composition selon la revendication 5, dans laquelle le polysorbate est présent dans une quantité comprise entre 50 et 200 mg/litre.

7. Composition selon l'une quelconque des revendications 1 à 6, dans laquelle la solution a une viscosité de moins de 2 Cp à température ambiante.

8. Procédé pour la préparation d'une composition d'aérosol à gouttelettes liquides telle que définie dans l'une quelconque des revendications 1 à 7, la composition ayant une activité biologique et une distribution de taille moléculaire IFN-γ connues et sélectionnées, lequel procédé comprenant :
la mise en place d'une solution aqueuse de IFN-γ humain contre une plaque ayant des ouvertures de taille définie, la solution ayant une activité biologique IFN-γ connue et sélectionnée, et contenant un agent stabilisant et un agent dispersant ;
le passage de force de la solution au travers desdites ouvertures dans des conditions efficaces pour former des gouttelettes aqueuses ayant
(a) un diamètre volumique moyen dans une gamme de taille de
(i) moins de 1 micron,
(ii) 1-3 microns,
(iii) 3-5 microns,
(iv) 5-10 microns,
(v) plus de 10 microns, ou
(vi) deux ou plus de i-v ;
(b) une activité biologique IFN-γ essentiellement la même que celle de la solution, et
(c) une distribution de taille moléculaire de IFN-γ essentiellement la même que celle de la solution.

9. Utilisation d'une composition d'aérosol à gouttelettes liquides selon l'une quelconque des revendications 1 à 7 pour la préparation d'une composition pharmaceutique pour traiter des désordres pulmonaires, des infections virales ou bactériennes, le cancer, ou des désordres fibrogènes.

10. Utilisation selon la revendication 9, pour traiter une pneumonie virale ou bactérienne, une tuberculose résistante aux médicaments, une maladie granulomateuse chronique, la mucoviscidose ou l'asthme.
